(19) Europäisches Patentamt

European Patent Office

Office européen des brevets

(11) **EP 0 845 439 B1**

(12) **EUROPÄISCHE PATENTSCHRIFT**

(45) Veröffentlichungstag und Bekanntmachung des Hinweises auf die Patenterteilung:
**23.02.2000 Patentblatt 2000/08**

(51) Int Cl.⁷: **C03B 23/09**, C03B 23/047, B01L 3/02, B41J 2/16, A61M 5/30

(21) Anmeldenummer: **97119775.1**

(22) Anmeldetag: **12.11.1997**

(54) **Glaskapillarrohr mit angeformter Einengung seines Innendurchmessers, Verfahren zum Herstellen eines derartigen Glaskapillarrohres und Vorrichtung zur Durchführung des Verfahrens**

Glass capillary tube having a shaped restriction of its internal diameter and process and apparatus for producing it

Tube capillaire de verre à diamètre interne réduit par façonnage et procédé et dispositif pour sa fabrication

(84) Benannte Vertragsstaaten:
**CH DE FR GB LI**

(30) Priorität: **28.11.1996 DE 19649335**

(43) Veröffentlichungstag der Anmeldung:
**03.06.1998 Patentblatt 1998/23**

(73) Patentinhaber: **SCHOTT-GERÄTE GmbH**
**65719 Hofheim a. Ts. (DE)**

(72) Erfinder:
• **Riege, Günter,**
**65195 Wiesbaden (DE)**

• **Günther, Siegfried,**
**65817 Eppstein (DE)**

(74) Vertreter: **Fuchs Mehler Weiss & Fritzsche**
**Patentanwälte**
**Abraham-Lincoln-Strasse 7**
**65189 Wiesbaden (DE)**

(56) Entgegenhaltungen:
**EP-A- 0 116 018      FR-A- 2 310 067**
**GB-A- 2 143 442**

**Beschreibung**

Die Erfindung betrifft ein Glaskapillarrohr mit dem Innendurchmesser (ID1), an dessen einem Ende eine Einengung seines Innendurchmessers, zusammengesetzt aus einer Übergangskontur (K) mit sich kontinuierlich verjüngendem Innendurchmesser auf einer bestimmten Länge (L1) und einem Endabschnitt mit konstantem verengten Innendurchmesser (ID2) angeformt ist.

Die Erfindung betrifft ferner ein Verfahren zum Herstellen eines Glaskapillarrohres, an dessen einem Ende eine Einengung seines Innendurchmessers, zusammengesetzt aus einer Übergangskontur mit sich kontinuierlich verjüngendem Innendurchmesser und einem Endabschnitt mit konstantem verengten Innendurchmesser angeformt ist.

Die Erfindung betrifft ferner eine Vorrichtung zur Durchführung eines solchen Verfahrens.

Glaskapillarrohre der vorgenannten Art finden in bekannter Weise beispielsweise Anwendung als Titrier- und Bürettenspitzen, bei Dosiergeräten und in der Lasertechnologie.

Es sind hauptsächlich zwei Verfahrensprinzipien bekannt, um derartige Glaskapillarrohre herzustellen.

Bei dem ersten Verfahrensprinzip erfolgt ein Ausziehen des erwärmten Glaskapillarrohres, bis der Innendurchmesser den gewünschten eingeengten Wert erreicht hat. Die Nachteile dieses Verfahrens bestehen hauptsächlich darin, daß

-    eine langgestreckte Übergangskontur entsteht, die einen unerwünscht langen Endbereich des Kapillarrohres bedingt,
-    die Übergangskontur nur relativ einfachen geometrischen Funktionen folgt, die bestimmte Anwendungen des Kapillarrohres nicht zuläßt,
-    der Toleranzbedarf für die Übergangskontur und den eingeengten Endabschnitt relativ groß ist.

Bei dem zweiten Verfahrensprinzip wird ein formgebender Kern in das Kapillarrohr eingebracht und dieses nach entsprechender Erwärmung praktisch auf den Kern aufgeschrumpft, der danach wieder aus dem Rohr entfernt wird. Die Nachteile dieses Verfahrensprinzipes bestehen hauptsächlich darin, daß

-    in dem Kapillarrohr Rückstände des Kernmaterials verbleiben, die bei bestimmten Anwendungen, z.B. in der Medizintechnik, unerwünscht sind,
-    es relativ aufwendig ist,
-    es ein zusätzliches, hohem Verschleiß unterworfenes Werkzeug benötigt,
-    die Übergangskontur auch nur relativ einfachen Funktionen folgt, da die Kernformfläche, damit das Verfahren wirtschaftlich durchzuführen ist, nur entsprechend einfache geometrische Formen aufweist, so daß die Einengung im Übergangsbereich eine übliche Trichterform annimmt.

Durch die US-A-4,675,043 ist ein Verfahren zum Herstellen eines dünnwandigen Glaskapillarrohres mit einer düsenförmigen Einengung des Innendurchmessers bekanntgeworden, bei dem das vertikal gehalterte, rotierende Glaskapillarrohr gleichmäßig in dem Bereich, in dem die Einengung ausgeformt werden soll, durch Brenner erwärmt wird. Sobald das Glas plastisch wird, reduzieren sich - ohne äußere mechanische Einwirkung - aufgrund physikalischer Gesetze sowohl der innere als auch der äußere Durchmesser der Austrittsöffnung gemessen und mit einem Sollwert verglichen wird. Abhängig von der Regelabweichung wird die Position der Brenner relativ zum Glasrohr, und damit das Maß der zugeführten Wärme, verstellt.

Dieses Verfahren ist mit Nachteil beschränkt auf sehr dünnwandige Glaskapillarrohre (5-12 x $10^{-2}$ mm), wie sie insbesondere für Tintenstrahldrucker benötigt werden. Sie können keinem hohen Innendruck ausgesetzt werden. Durch die Regelung des Innendurchmessers allein über die Position der Brenner relativ zum Glaskapillarrohr, d.h. über die Größe der Erwärmung, ist diese Regelung aufgrund der trägen Wärmeeffekte ebenfalls sehr träge und bedingt daher mit Nachteil große Toleranzen hinsichtlich des Durchmessers der angeformten Austrittsöffnung. Weiterhin wird lediglich ein Durchmesserwert geregelt, nicht jedoch der Verlauf der Übergangskontur, der sehr temperaturabhängig ist und damit entsprechend den großen Toleranzen hinsichtlich der Wärmezuführ durch die Brenner ebenfalls großen Toleranzen unterworfen ist.

Der Erfindung liegt die Aufgabe zugrunde, ein Glaskapillarrohr der eingangs bezeichneten Art zu schaffen, dessen Übergangskontur und eingeengter Endabschnitt innerhalb geringer Toleranzen, auch anspruchsvollen geometrischen Funktionen folgend, rückstandsfrei herstellbar ist.

Die Lösung dieser Aufgabe gelingt für das eingangs bezeichnete Glaskapillarrohr erfindungsgemäß dadurch, daß der Verlauf der Übergangskontur (K) des Innendurchmessers durch die Funktion

$$K=F(Li) = (((1 +cos((\pi/L1)*L))*(ID1-ID2)/4))+ID2/2$$

bestimmt ist, mit L = Li*L1 und einem zulässigen Wertebereich von Li zwischen O und +1.

Hinsichtlich des eingangs bezeichneten Verfahrens zum Herstellen des vorgenannten Glaskapillarrohres gelingt die Lösung der Aufgabe erfindungsgemäß mit den Schritten:

- Gleichmäßiges Erwärmen eines um seine Längsachse mit konstanter Umlaufgeschwindigkeit rotierenden Glaskapillarrohres in dem Bereich, in dem die Einengung angeformt wird,
- Unterstützen der Ausformung der Einengung bei Erreichen der Plastizität des Glaskapillarrohres in dem erwärmten Bereich durch eine Formrolle, die im rechten Winkel zur Glaskapillarrohrachse verfahrbar ist,
- Zyklisches Messen zumindest des Innendurchmessers in der anzuformenden Einengung an verschiedenen, axial beabstandeten Punkten,
- Vergleichen der Meßwerte für jeden Punkt mit gespeicherten Sollwerten, die aus einer vorgegebenen Funktion für den Verlauf der Übergangskontur für den jeweiligen Punkt berechnet werden, wobei der Verlauf der Übergangskontur (K) des Innendurchmessers durch die Funktion

$$K=F(Li)=(((1+\cos((\pi/L1)*L))*(ID1-ID2)/4))+ID2/2$$

bestimmt ist,
   mit L=Li*L1 und einem zulässigen Wertebereich von Li zwischen 0 und +1.
- Einstellen des Vorschubes der Formrolle, abhängig von dem Vergleich, bis der jeweilige Meßwert mit dem zugehörigen Sollwert übereinstimmt.

Hinsichtlich der zugehörigen Vorrichtung erfolgt die Lösung der Aufgabe gemäß der Erfindung mit:

- einer Brenneranordnung zum gleichmäßigen Erwärmen eines um seine Längsachse mit konstanter Umlaufgeschwindigkeit rotierenden Glaskapillarrohres in dem Bereich, in dem die Einengung anzuformen ist,
- einer Formrolle zum Unterstützen der Ausformung der Einengung bei Erreichen der Plastizität des Glaskapillarrohres in dem erwärmten Bereich,

   die im rechten Winkel zur Glaskapillarrohrachse verfahrbar ist,

- einer Meßeinrichtung mit einer Meßkamera zum zyklischen Messen, zumindest des Innendurchmessers in der anzuformenden Einengung an verschiedenen, axial beabstandeten Punkten,
- einer Logik-Stufe zum Vergleichen der Meßwerte für jeden Punkt mit gespeicherten Sollwerten, die aus einer vorgegebenen Funktion für den Verlauf der Übergangskontur für den jeweiligen Punkt berechnet werden,
- Einrichtungen zum Einstellen des Vorschubes der Formrolle, abhängig von dem Vergleich in der Logik-Stufe, bis der jeweilige Meßwert mit dem zugehörigen Sollwert übereinstimmt,

oder alternativ
mit:

- einer Logik-Stufe, in der empirisch gewonnene Daten, die den Verlauf der gewünschten Übergangskontur beschreiben, gespeichert sind,
- Einrichtungen zum Einstellen des Vorschubes der Formrolle, abhängig von diesen Daten.

Mittels der Erfindung ist ein Glaskapillarrohr mit angeformter Einengung herstellbar, das, weil ohne Kern hergestellt, rückstandsfrei ist, und bei dem der Verlauf der Übergangskontur mit dem eingeengten Endabschnitt anspruchsvollen geometrischen Funktionen folgend, innerhalb geringer Toleranzen ausbildbar ist, weil die Vorschub-Steuerung bzw. -Regelung der Formrolle sehr genau und trägheitsarm der Sollkurve folgt. Auf diese Weise ist insbesondere eine Übergangskontur ausbildbar, die eine ausgeprägte Düsenfunktion bewirkt, so daß das erfindungsgemäße Kapillarrohr als nadellose Injektionsspritze in der Medizintechnik einsetzbar ist.

Durch die äußere mechanische Unterstützung mittels der Formrolle lassen sich auch Einengungen in relativ dickwandigen Kapillarrohren ausformen, die relativ hohen Innendrücken aussetzbar sind.

Die Verfahren lassen sich besonders vorteilhaft durchführen, wenn der Vorschub der Formrolle hinsichtlich seiner Größe bzw. der Geschwindigkeit verstellt wird. Bei dieser Ausgestaltung der Erfindung läßt sich besonders feinfühlig die gewünschte Übergangskontur nachformen.

Ein besonders wirtschaftlicher Verfahrensablauf ist gemäß einer Weiterbildung der Erfindung erzielbar, wenn das Kapillarrohr mittig erwärmt wird, die Formrolle unter Erzeugung von zwei spiegelsymmetrischen Einengungen mittig in Eingriff mit dem Glaskapillarrohr gebracht wird, und später eine mittige Trennung erfolgt.

Weitere Ausgestaltungen der Erfindung ergeben sich anhand von in der Beschreibung dargestellten Ausführungsbeispielen der Erfindung.

Es zeigen:

Fig. 1 ein Kapillarrohr mit der erfindungsgemäß angeformten Einengung,

Fig. 2 den Verlauf der Übergangskontur der Einengung als geometrische Kurve in einem kartesischen Koordinatensystem,

Fig. 3 eine Prinzipdarstellung des Fertigungsaufbaues,

Fig. 4 die Regel- bzw. Steuereinrichtung für den Anformvorgang,

Fig. 5 den Verlauf der Kontur der Einengung bei einer symmetrisch mittigen Anformung.

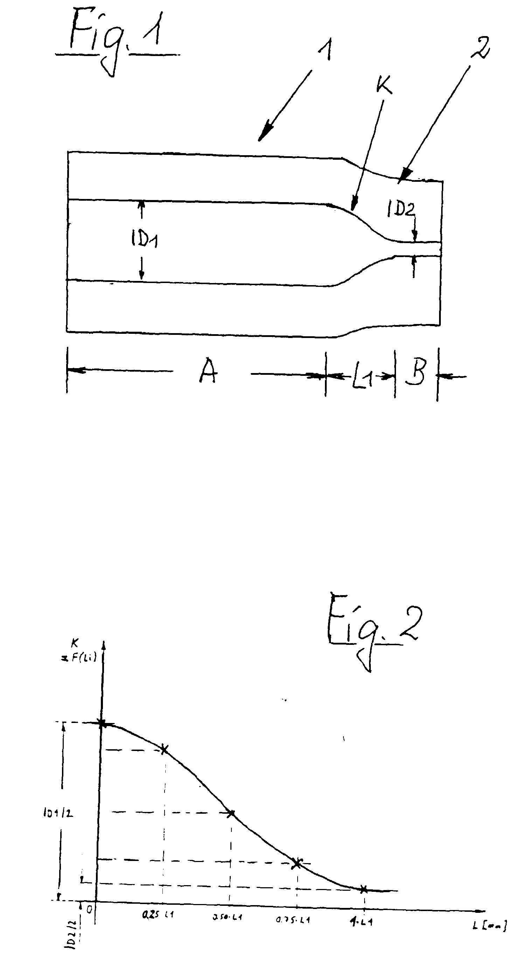

Die Fig. 1 zeigt ein erfindungsgemäß ausgebildetes Glaskapillarrohr 1 mit seinem Innendurchmesser ID1, der über die Länge A konstant ist. Die Länge A richtet sich dabei nach der vorgesehenen Anwendung des Kapillarrohres.

Der Außendurchmesser (AD) des Kapillarrohres liegt typischerweise im Bereich zwischen 4,0 mm und 9,0 mm, sein Innendurchmesser (ID1) im Bereich zwischen 1,0 mm und 3,0 mm und die Wandstärke im Bereich zwischen 1,3 mm und 3,0 mm.

An seinem Ende 2 besitzt das Kapillarrohr eine angeformte Einengung seines Innendurchmessers ID1. Diese Einengung setzt sich zusammen aus einer Übergangskontur K der Länge L1 mit sich kontinuierlich verjüngendem Innendurchmesser und einem Endabschnitt B mit konstantem verengten Innendurchmesser ID2. Die Länge L1 der Übergangskontur liegt typischerweise im Bereich zwischen 4,0 mm und 8,0 mm, und der eingeengte Innendurchmesser ID2 im Bereich zwischen 0,1 mm und 2,5 mm. Für die Durchmesserwerte ID1 und ID2 gilt weiterhin die Bedingung: ID1 - ID2 größer/gleich 0,5 mm.

Der Verlauf der Übergangskontur K ist durch die Größen L1, ID1 und ID2 spezifiziert. Dieser Übergang vom Abschnitt A mit konstantem Innendurchmesser ID1 zum Abschnitt B mit eingeengten konstanten Innendurchmesser ID2 hat einen Verlauf, wie er in Fig. 2 in einem kartesischen Koordinatensystem dargestellt ist. Der Verlauf der Kontur K = F(L1) entspricht dabei der Funktion:

$$K=F(Li)=(((1+\cos((\pi/L1)*L))*(ID1-ID2)/4))+ID2/2$$

wobei $L = Li*L1$ und der zulässige Wertebereich von Li zwischen 0 und +1 liegen darf.

Es ergeben sich für die Eckwerte des Wertebereiches von Li gemäß Funktion K = F(Li):

Für Li = 0       [L = 0]:       Kontur K = F(Li) = ID1/2

Für Li = 1       [L = L1]:       Kontur K = F(Li) = ID2/2

Dazwischen ist die Funktion stetig, wie später noch zu erläutern sein wird. Ebenso werden die in Fig. 2 dargestellten Zwischenwerte später noch erläutert.

Die in den Fig. 1 und 2 dargestellte erfindungsgemäße Übergangskontur bedingt besondere Vorteile. Sie bewirkt insbesondere einen Düseneffekt und ermöglicht es, das Kapillarrohr nach Fig. 1 als nadellose Injektionsspritze, insbesondere im medizinischen Bereich, einzusetzen. Da, wie noch zu erläutern sein wird, kein formgebender Kern bei der Herstellung des Kapillarrohres bzw. der Anformung der Einengung verwendet wird, ist das Produkt nach Fig. 1 völlig rückstandsfrei und daher auch in dieser Hinsicht gerade für medizinische Anwendungen geeignet. Hinzu kommt mit Vorteil, daß dieses Produkt mit sehr engen Toleranzen herstellbar ist.

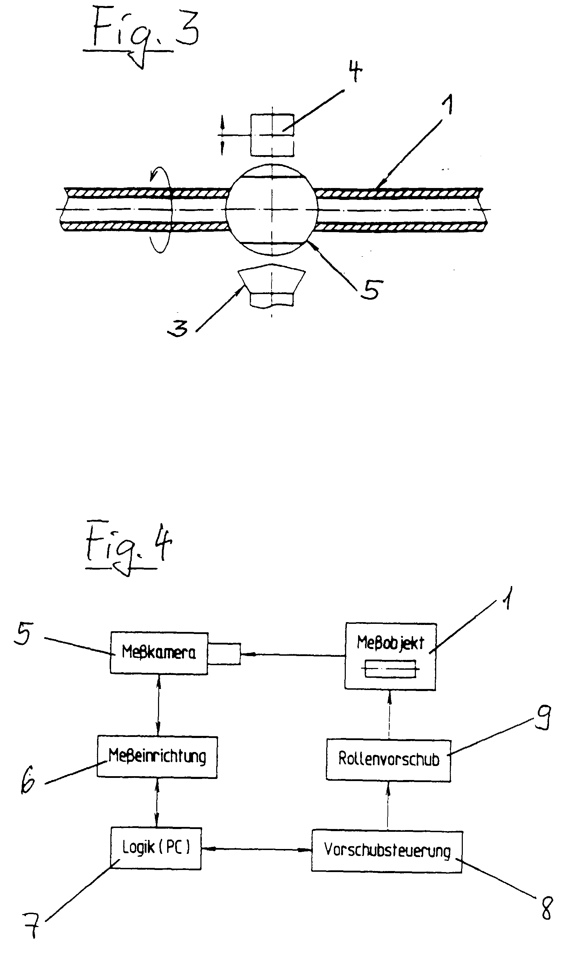

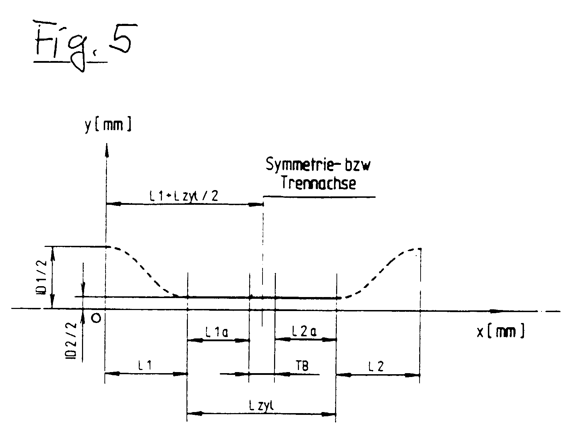

Anhand der Vorrichtung nach den Fig. 3 und 4 soll nunmehr das Herstellen des Glaskapillarrohres nach Fig. 1 erläutert werden.

Die Fig. 3 zeigt dabei eine Prinzipdarstellung des Fertigungsaufbaues. Ein um seine Längsachse mit konstanter Umlaufgeschwindigkeit rotierendes Glaskapillarrohr 1 wird in dem Bereich, wo die Einengung angeformt werden soll, mittels einer Brenneranordnung 3 gleichmäßig erwärmt.

Grundsätzlich ist es denkbar, das Kapillarrohr an einem Ende einzuengen. Vorteilhafter ist es jedoch, entsprechend der Prinzipdarstellung nach Fig. 5 das Kapillarrohr mittig einzuengen und längs der Symmetrieachse anschließend mit bekannten Trennhilfen zu trennen, so daß in einem Fertigungsschritt zwei Produkte nach Fig. 1 erhältlich sind. Die Einengung setzt sich in diesem Fall zusammen aus der Länge L1 der einen Übergangskontur und der Länge L2 der dazu symmetrischen anderen Übergangskontur, sowie der Länge Lzyl, innerhalb der der eingeengte Innendurchmesser ID2 konstant ist, mit

$$Lzy1 = L1a + TB + L2a$$

wobei

L1a =   ID2-Konstante Soll-Länge der ersten Einengung

TB =   Breite einer Trennhilfe

L2a =   ID2-Konstante Soll-Länge der zweiten, symmetrischen Einengung ist.

Bei Erreichen seiner Plastizität beginnt sich aufgrund der charakteristischen Eigenschaften von Glas der innere Durchmesser zunächst ohne mechanische Unterstützung von außen zu verjüngen. Um eine durch L1, ID1 und ID2 spezifizierte Übergangskontur zu erhalten, unterstützt eine symmetrische Formrolle 4, die im rechten Winkel zur Glasrohrachse verfahren werden kann, die Ausformung der Kontur. Das Einengen, d.h. die Veränderung des Innendurchmessers, wird dabei von einer berührungslosen Meßeinrichtung mit Meßkamera 5 zyklisch überwacht, mit einer Zykluszeit <40 ms.

Das rechtwinklige kartesische Koordinatensystem der Meßeinrichtung 5 liegt in X-Richtung deckungsgleich mit der Längsachse der Kapillarröhre 1 und wird selbständig vom Meßsystem durch Abstandsmessung der sich gegenüberliegenden Kanten des Innendurchmessers ID1 zu ID1/2 ermittelt und gegebenenfalls nachgeführt. Damit wird erreicht, daß Formfehler der Kapillarröhre - Ovalität, Inhomogenität der Wandstärke und Konzentrizität ID/AD nach DIN ISO 1101 - keinen Einfluß auf die Symmetrie der Längsachse der Röhre gegenüber der Kontur der Einengung haben.

Der Nullpunkt der X-Achse liegt in Höhe des Punktes der Einengung, wo die Abweichung vom Innendurchmesser IDI beginnt, d.h. der Funktionswert y = IDI/2 ist (siehe Fig. 2).

Die Ausformung der Kontur erfolgt dabei mit der in Fig. 4 dargestellten elektronischen Regelanordnung. Die Meßkamera 5 der Meßeinrichtung 6 überwacht zyklisch das Meßobjekt, die Glaskapillarröhre 1, hinsichtlich ihres Innendurchmessers ID, und zwar an verschiedenen Punkten "i" der Länge L1 (Fig. 2) bzw. L2 (Fig. 5). Die Meßwerte werden einer Logik 7 zugeführt, die vorzugsweise ein Mikroprozessor bzw. ein PC ist. In dieser Logik 7 sind als Sollwerte die errechneten Funktionswerte für die vorgenannten Meßpunkte "i" entlang der Übergangsbereiche L1 und L2 gespeichert.

Da die charakteristischen Eigenschaften von Glas bei Erwärmung keine sprunghaften Formveränderungen erwarten lassen, kann davon ausgegangen werden, daß die in Fig. 2 dargestellte Funktion K = F(Li) im Intervall Li = 0 bis Li = 1 stetig ist.

Daher kann davon ausgegangen werden, daß, um die Konturtreue des Werkstückes mit der Funktion K = F(Li) sicherzustellen, es ausreicht, mindestens für Li = 0,25, 0,50, 0,75 den entsprechenden Funktionswert K = F(Li) zu berechnen.

Die Meßwerte für den Innendurchmesser, d.h. die IST-Werte, werden in der Stufe 7 fortlaufend mit den errechneten Funktionswerten, d.h. den zugehörigen Sollwerten, verglichen, und abhängig von einer festgestellten Differenz werden über eine Vorschubsteuerung 6 und einen Rollenvorschub 9 die Größe des Vorschubes, die Vorschubrichtung und die Vorschubgeschwindigkeit der Formrolle 4 derart gesteuert, daß sie die Kontur der Einengung entsprechend der vorgegebenen Funktion nach Fig. 2 bzw. Fig. 5 mit spezifizierten Toleranzen formen.

Sobald sich der Kapillarinnendurchmesser durch die Flammeinwirkung des Brenners 3 in der für Glas charakteristischen Weise ohne mechanische Einwirkung von außen zu verengen beginnt, wird automatisch dieser elektronische "Dialog" zwischen Meßeinrichtung 6, Logik 7 und der Vorschub-Steuereinheit 8, 9 gestartet, der bis Fertigungsende aufrechterhalten bleibt.

Die Formrolle 4 hat dabei eine symmetrische plane Andrückfläche, deren Breite vorab im Versuch zu ermitteln ist.

Im Ausführungsbeispiel sind gemäß der Fig. 2 drei Zwischenpunkte für einen SOLL-IST-Wertvergleich vorgesehen. Es ist verständlich, daß auch eine andere Zahl von Zwischenpunkten gewählt werden kann. Die Praxis hat gezeigt, daß mit den beschriebenen drei Meßpunkten auf einfache Weise die vorgegebene Kontur mit ausreichender Genauigkeit nachgeformt werden kann.

Der Fertigungsprozeß gemäß der Fig. 4 basiert auf einem fortwährenden Vergleich zwischen IST- und SOLL-Werten, d.h. enthält eine Regelung. Es ist auch möglich, eine Steuerung vorzusehen. In diesem Fall überwacht die Meßeinrichtung 5, 6 nicht zyklisch den Fertigungsprozeß sondern ermittelt einmalig bei Start des Einengvorganges die Form- und Lägetoleranzen der Kapillarröhre (= Ovalität und Konzentrizität bezüglich ID/AD spezifiziert gemäß DIN ISO 1101).

Vorschub, dessen Richtung und Verfahrgeschwindigkeiten der Formrolle 4 werden in der Stufe 7 über eine Funktion berechnet, deren Stammdaten sich aus Vorversuchen der Fertigung und deren Analyse dort gewonnener Meßwerte, die Abhängigkeiten zwischen Form- und Lägetoleranzen der Kapillarröhre zu deren spezifizierter Konturtreue und Maßhaltigkeit gründen, zusammensetzen.

Dieses Verfahren stellt keine hohen Anforderungen hinsichtlich Meßzykluszeit (ca. 1 Sekunde). Es kann dort Anwendung finden, wo Toleranzen des verjüngten Innendurchmessers mehrere Zehntel Millimeter betragen können.

Die Fertigungseinrichtung nach den Figuren 3 und 4 sowie das zugehörige Verfahren ist nicht auf die Nachformung der Einengung nach den, Kurvenverlauf gemäß der Fig. 2 beschränkt. Es können auch andere Funktionen nachgeformt werden, wenn entsprechend berechnete Sollwerte in der Stufe 7 abgelegt sind und die Meßeinrichtung 5, 6 die die Funktion bestimmenden Werte mißt.

## Patentansprüche

1. Glaskapillarrohr (1) mit dem Innendurchmesser (ID1), an dessen einem Ende (2) eine Einengung seines Innendurchmessers, zusammengesetzt aus einer Übergangskontur (K) mit sich kontinuierlich verjüngendem Innendurchmesser auf einer bestimmten Länge (L1) und einem Endabschnitt (B) mit konstantem verengten Innendurchmesser (ID2), angeformt ist, dadurch gekennzeichnet, daß der Verlauf der Übergangskontur (K) des Innendurchmessers durch die Funktion

$$K=F(Li)=(((1+\cos((\pi/L1)*L))*(ID1-ID2)/4))+ID2/2$$

   bestimmt ist,
   mit L = Li*L1 und einem zulässigen Wertebereich von Li zwischen 0 und +1.

2. Verfahren zum Herstellen eines Glaskapillarrohres nach Anspruch 1, an dessen einem Ende eine Einengung seines Innendurchmessers (ID1), zusammengesetzt aus einer Übergangskontur (K) mit sich kontinuierlich verjüngendem Innendurchmesser auf einer bestimmten Länge (L1) und einem Endabschnitt (B) mit konstantem verengten Innendurchmesser (ID2), angeformt ist, mit den Schritten:

   - Gleichmäßiges Erwärmen eines um seine Längsachse mit konstanter Umlaufgeschwindigkeit rotierenden Glaskapillarrohres in dem Bereich, in dem die Einengung angeformt wird,
   - Unterstützen der Ausformung der Einengung bei Erreichen der Plastizität des Glaskapillarrohres in dem erwähnten Bereich durch eine Formrolle, die im rechten Winkel zur Glaskapillarrohrachse verfahrbar ist,
   - Zyklisches Messen zumindest des Innendurchmessers in der anzuformenden Einengung an verschiedenen, axial beabstandeten Punkten,
   - Vergleichen der Meßwerte für jeden Punkt mit gespeicherten Sollwerten, die aus einer vorgegebenen Funktion für den Verlauf der Übergangskontur für den jeweiligen Punkt berechnet werden, wobei der Verlauf der Übergangskontur (K) des Innendurchmessers durch die Funktion

$$K = F(Li)=(((1+\cos((\pi/L1)*L))*(ID1-ID2)/4))+ID2/2$$

   bestimmt ist,
   mit L=Li*L1 und einem zulässigen Wertebereich von Li zwischen 0 und +1,
   - Einstellen des Vorschubes der Formrolle, abhängig von dem Vergleich, bis der jeweilige Meßwert mit dem zugehörigen Sollwert übereinstimmt.

3. Verfahren nach Anspruch 2, dadurch gekennzeichnet, daß das Kapillarrohr mittig erwärmt wird, die Formrolle unter Erzeugung von zwei spiegelsymmetrischen Einengungen mittig in Eingriff mit dem Glaskapillarrohr gebracht wird, und später eine mittige Trennung erfolgt.

4. Vorrichtung zur Durchführung der Verfahren nach Anspruch 2 oder 3, mit:

   - einer Brenneranordnung (3) zum gleichmäßigen Erwärmen eines um seine Längsachse mit konstanter Umlaufgeschwindigkeit rotierenden Glaskapillarrohres (1) in dem Bereich, in dem die Einengung anzuformen ist,
   - einer Formrolle (4) zum Unterstützen der Ausformung der Einengung bei Erreichen der Plastizität des Glaskapillarrohres (1) in dem erwärmten Bereich, die im rechten Winkel zur Glaskapillarrohrachse verfahrbar ist,

-   einer Meßeinrichtung (6) mit einer Meßkamera (5) zum zyklischen Messen, zumindest des Innendurchmessers in der anzuformenden Einengung an verschiedenen, axial beabstandeten Punkten,
-   einer Logik-Stufe (7) zum Vergleichen der Meßwerte für jeden Punkt mit gespeicherten Sollwerten, die aus einer vorgegebenen Funktion für den Verlauf der Übergangskontur für den jeweiligen Punkt berechnet werden,
-   Einrichtungen (8, 9) zum Einstellen des Vorschubes der Formrolle (4), abhängig von dem Vergleich in der Logik-Stufe (7), bis der jeweilige Meßwert mit dem zugehörigen Sollwert übereinstimmt.

5.  Vorrichtung zur Durchführung des Verfahrens nach Anspruch 2 oder 3,
    mit:

    -   einer Brenneranordnung (3) zum gleichmäßigen Erwärmen eines um seine Längsachse mit konstanter Umlaufgeschwindigkeit rotierenden Glaskapillarrohres (1) in dem Bereich, in dem die Einengung anzuformen ist,
    -   einer Formrolle (4) zum Unterstützen der Ausformung der Einengung bei Erreichen der Plastizität des Glaskapillarrohres (1) in dem erwärmten Bereich, die im rechten Winkel zur Glaskapillarrohrachse verfahrbar ist,
    -   einer Logik-Stufe (7), in der empirisch gewonnene Daten, die den Verlauf der gewünschten Übergangskontur beschreiben, gespeichert sind,
    -   Einrichtungen (8, 9) zum Einstellen des Vorschubes der Formrolle (4), abhängig von diesen Daten.

6.  Vorrichtung nach Anspruch 4 oder Anspruch 5, dadurch gekennzeichnet, daß die Formrolle (4) symmetrisch mit einer planen Andrückfläche vorgegebener Breite ausgebildet ist.

7.  Vorrichtung nach Anspruch 4 oder 6, dadurch gekennzeichnet, daß die Meßkamera (5) ein sich selbsttätig mittig auf die Längsachse des Kapillarrohres einstellendes Koordinatensystem besitzt.

8.  Vorrichtung nach Anspruch 5 oder 6, dadurch gekennzeichnet, daß zusätzlich eine Meßeinrichtung (5, 6) vorgesehen ist, die bei Beginn des Einengvorganges die Form- und Lagetoleranzen des Kapillarrohres (1) ermittelt, die als Korrekturwerte der Logik-Stufe (7) zuführbar sind.

## Claims

1.  Glass capillary tube (1) having an internal diameter (ID1) at one end (2) of which a constriction of its internal diameter is formed, composed of a transition contour (K) with a continuously tapering internal diameter over a specific length (L1) and an end section (B) with a constant narrowed internal diameter (ID2), characterized in that the course of the transition contour (K) of the internal diameter is determined by the function

$$K = F(Li) = (((1+\cos((\pi/L1)*L))*(ID1-ID2)/4))+ID2/2,$$

    where L = Li*L1 and Li has a permissible range between 0 and +1.

2.  Process for producing a glass capillary tube according to Claim 1 at one end of which a constriction of its internal diameter (ID1) is formed, composed of a transition contour (K) with a continuously tapering internal diameter over a specific length (L1) and an end section (B) with a constant narrowed internal diameter (ID2), having the steps:

    -   Uniformly heating a glass capillary tube, rotating at a constant speed of revolution about its longitudinal axis, in the region in which the constriction is formed,
    -   Supporting the shaping of the constriction, when the plasticity of the glass capillary tube is reached in the aforementioned region, by means of a shaping roller, which can be moved at right angles to the axis of the glass capillary tube,
    -   Cyclically measuring at least the internal diameter in the constriction to be formed at different, axially spaced points,
    -   Comparing the measured values for each point with stored desired values, which are calculated for the respective point from a prescribed function for the course of the transition contour, the course of the transition contour (K) of the internal diameter being determined by the function

$$K = F(Li) = (((1+\cos((\pi/L1)*L))*(ID1-ID2)/4))+ID2/2,$$

where L = Li*L1 and Li has a permissible range between 0 and +1,

- Adjusting the advance of the shaping tube, as a function of the comparison, until the respective measured value agrees with the associated desired value.

3. Process according to Claim 2, characterized in that the capillary tube is heated at its centre, the shaping roller is moved so as to act on the centre of the glass capillary tube, producing two mirror-symmetrical constrictions, and division at the centre being carried out subsequently.

4. Apparatus for carrying out the process according to Claim 2 or 3, having:

- a burner arrangement (3) for uniformly heating a glass capillary tube (1), rotating at a constant speed of revolution about its longitudinal axis, in the region in which the constriction is to be formed,
- a shaping roller (4) for supporting the shaping of the constriction, when the plasticity of the glass capillary tube (1) is reached in the heated region, which can be moved at right angles to the axis of the glass capillary tube,
- a measuring device (6) having a measuring camera (5) for cyclically measuring at least the internal diameter in the constriction to be formed at different, axially spaced points,
- a logic stage (7) for comparing the measured values for each point with stored desired values, which are calculated for the respective point from a prescribed function for the course of the transition contour,
- devices (8, 9) for adjusting the advance of the shaping roller (4), as a function of the comparison in the logic stage (7), until the respective measured value agrees with the associated desired value.

5. Apparatus for carrying out the process according to Claim 2 or 3, having:

- a burner arrangement (3) for uniformly heating a glass capillary tube (1), rotating at a constant speed of revolution about its longitudinal axis, in the region in which the constriction is to be formed,
- a shaping roller (4) for supporting the shaping of the constriction, when the plasticity of the glass capillary tube (1) is reached in the heated region, which can be moved at right angles to the axis of the glass capillary tube,
- a logic stage (7), in which data which have been obtained empirically and which describe the course of the desired transition contour are stored,
- devices (8, 9) for adjusting the advance of the shaping roller (4) as a function of these data.

6. Apparatus according to Claim 4 or Claim 5, characterized in that the shaping roller (4) is of symmetrical design with a planar pressure face of prescribed width.

7. Apparatus according to Claim 4 or 6, characterized in that the measuring camera (5) has a co-ordinate system which automatically sets itself centrally with respect to the longitudinal axis of the capillary tube.

8. Apparatus according to Claim 5 or 6, characterized in that a measuring device (5, 6) is additionally provided which, at the start of the constricting process, determines the tolerances of shape and position of the capillary tube (1), which can be fed as correction values to the logic stage (7).

**Revendications**

1. Tube capillaire en verre (1) ayant un diamètre intérieur (ID1), à l'une des extrémités (2) duquel est façonné un rétrécissement de son diamètre intérieur, composé d'un contour de transition (K) ayant un diamètre intérieur qui se réduit continuellement sur une longueur (L1) donnée et une section finale (B) ayant un diamètre intérieur (ID2) qui se rétrécit constamment, caractérisé par le fait que le tracé du contour de transition (K) du diamètre intérieur est déterminé par la fonction

$$K = F(Li) = (((1 + \cos((\pi/L1) * L)) * (ID1 - ID2)/4)) + ID2/2,$$

avec L = Li * L1 et une plage autorisée des valeurs de Li entre 0 et +1.

2. Procédé pour fabriquer un tube capillaire en verre selon la revendication 1, à l'une des extrémités duquel est façonné un rétrécissement de son diamètre intérieur (ID1), composé d'un contour de transition (K) ayant un diamètre intérieur qui se réduit continuellement sur une longueur (L1) donnée et une section finale (B) ayant un

8

diamètre intérieur (ID2) qui se rétrécit constamment, comprenant les étapes suivantes :

- échauffement régulier d'un tube capillaire en verre tournant à une vitesse circonférentielle constante autour de son axe longitudinal dans la zone où sera façonné le rétrécissement,
- assistance au façonnage du rétrécissement en atteignant la plasticité du tube capillaire en verre dans la zone échauffée par un rouleau de façonnage qui peut se déplacer en angle droit par rapport à l'axe du tube capillaire en verre,
- mesure cyclique au moins du diamètre intérieur dans le rétrécissement à façonner en différents points séparés dans le sens axial,
- comparaison des valeurs mesurées de chaque point avec des valeurs de consigne mémorisées qui sont calculées à partir d'une fonction prédéfinie pour le tracé du contour de transition pour chacun des points, le tracé du contour de transition (K) du diamètre intérieur étant déterminé par la fonction

$$K = F(Li) = (((1 + \cos ((\pi/L1) * L)) * (ID1 - ID2)/4)) + ID2/2,$$

avec $L = Li * L1$ et une plage autorisée des valeurs de Li entre 0 et +1,
- réglage de l'avance du rouleau de façonnage en fonction de la comparaison jusqu'à ce que la valeur mesurée corresponde à la valeur de consigne associée.

3. Procédé selon la revendication 2, caractérisé par le fait que le tube capillaire est échauffé au centre, que le rouleau de façonnage est amené en prise avec le tube capillaire en verre au centre en produisant deux rétrécissements symétriques en miroir et qu'il se produit plus tard une séparation au centre.

4. Dispositif pour réaliser le procédé selon la revendication 2 ou 3, comprenant :

- un arrangement de brûleur (3) pour échauffer simultanément un tube capillaire en verre (1), tournant à une vitesse circonférentielle constante autour de son axe longitudinal, dans la zone dans laquelle doit être façonné le rétrécissement,
- un rouleau de façonnage (4) pour assister le façonnage du rétrécissement en atteignant la plasticité du tube capillaire en verre (1) dans la zone échauffée, lequel peut se déplacer en angle droit par rapport à l'axe du tube capillaire en verre,
- un dispositif de mesure (6) muni d'une caméra de mesure (5) pour la mesure cyclique au moins du diamètre intérieur dans le rétrécissement à façonner en différents points séparés dans le sens axial,
- un étage logique (7) pour comparer les valeurs mesurées de chaque point avec des valeurs de consigne mémorisées qui sont calculées à partir d'une fonction prédéfinie pour le tracé du contour de transition pour chacun des points,
- des dispositifs (8, 9) pour régler l'avance du rouleau de façonnage (4) en fonction de la comparaison dans l'étage logique (7), jusqu'à ce que la valeur mesurée corresponde à la valeur de consigne associée.

5. Dispositif pour réaliser le procédé selon la revendication 2 ou 3, comprenant :

- un arrangement de brûleur (3) pour échauffer simultanément un tube capillaire en verre (1), tournant à une vitesse circonférentielle constante autour de son axe longitudinal, dans la zone dans laquelle doit être façonné le rétrécissement,
- un rouleau de façonnage (4) pour assister le façonnage du rétrécissement en atteignant la plasticité du tube capillaire en verre (1) dans la zone échauffée, lequel peut se déplacer en angle droit par rapport à l'axe du tube capillaire en verre,
- un étage logique (7) dans lequel sont mémorisées des données acquises de manière empirique et qui décrivent le tracé du contour de transition souhaité,
- des dispositifs (8, 9) pour régler l'avance du rouleau de façonnage (4) en fonction de ces données.

6. Dispositif selon la revendication 4 ou la revendication 5, caractérisé par le fait que le rouleau de façonnage (4) est de forme symétrique avec une surface d'appui plane de largeur donnée.

7. Dispositif selon la revendication 4 ou 6, caractérisé par le fait que la caméra de mesure (5) possède un système de coordonnées qui se règle automatiquement au centre de l'axe longitudinal du tube capillaire.

8. Dispositif selon la revendication 5 ou 6, caractérisé par le fait qu'un dispositif de mesure (5, 6) supplémentaire est prévu, lequel détermine au début de l'opération de rétrécissement les tolérances de forme et de position du tube capillaire (1) qui pourront être amenées comme valeurs de correction à l'étage logique (7).

Fig. 1

Fig. 2

Fig. 3

Fig. 4

Fig. 5